# EUROPEAN PATENT SPECIFICATION

(11) **EP 3 901 264 B1**
(45) Date of publication and mention of the grant of the patent: **15.07.2026**
(21) Application number: 19891918.5
(22) Date of filing: 05.12.2019
(51) Int. Cl.: C12N 15/12, C12Q 1/6851, G01N 33/53

(54) **METHOD FOR PREDICTING EFFICACY OF RHEUMATOID ARTHRITIS THERAPEUTIC AGENT AND BIOMARKER USED THEREIN**
VERFAHREN ZUR VORHERSAGE DER WIRKSAMKEIT EINES THERAPEUTISCHEN MITTELS GEGEN RHEUMATOIDE ARTHRITIS UND DARIN VERWENDETER BIOMARKER
PROCÉDÉ DE PRÉDICTION DE L' EFFICACITÉ D' UN AGENT THÉRAPEUTIQUE CONTRE LA POLYARTHRITE RHUMATOÏDE ET BIOMARQUEUR UTILISÉ DANS LE CADRE DUDIT PROCÉDÉ

(30) Priority: 07.12.2018 US 201862776905 P
(43) Date of publication of application: 27.10.2021
(73) Proprietor: DNA Chip Research Inc., Tokyo, 1050022 (JP); Keio University, Tokyo, 108-8345 (JP); Saitama Medical University, Iruma-gun, Saitama 350-0495 (JP)
(72) Inventor: NAKAMURA Seiji, Tokyo 105-0022 (JP); UEDA Yumi, Tokyo 105-0022 (JP); ISHIZAWA Yohei, Tokyo 105-0022 (JP); HIRAYAMA Madoka, Tokyo 105-0022 (JP); MATOBA Ryo, Tokyo 105-0022 (JP); TAKEUCHI Tsutomu, Tokyo 160-8582 (JP); YAMAOKA Kunihiro, Tokyo 160-8582 (JP); AMANO Koichi, Iruma-gun, Saitama 350-0495 (JP)
(74) Representative: JWP Patent & Trademark Attorneys
(86) International application number: PCT/JP2019/047645
(87) International publication number: WO 2020/116567

(56) References cited:
- WO-A1-2017/125402
- WO-A1-2017/125402
- WO-A2-2008/150491
- JP-A- 2009 092 508
- US-A1- 2016 194 709
- US-B2- 8 092 998
- TAKEUCHI, TSUTOMU ET AL.: "Reactivity and DNA chip analysis of biologics for rheumatoid arthritis", JAPANESE JOURNAL OF ALLERGOLOGY, vol. 54, no. 8- 9, 30 September 2005 (2005-09-30), pages 927, XP055713987

## Description

### FIELD OF THE INVENTION

The present invention pertains to a method for predicting the efficacy of a rheumatoid arthritis therapeutic agent, and to a biomarker therein. Specifically, the present invention pertains to a method for predicting the efficacy of a rheumatoid arthritis therapeutic agent by measuring and standardizing the expression levels of a biomarker gene according to the present invention in blood taken from a subject before and after therapy, comparing the change in expression level, and estimating a future rheumatoid arthritis activity index for the subject. The present invention also pertains to a method for estimating a numerical value for an index associated with rheumatoid arthritis in a subject.

### BACKGROUND OF THE INVENTION

Rheumatoid arthritis ("RA"), a typical rheumatic disease, is a systemic connective tissue disease primarily manifesting as chronic polyarthritis, and a type of autoimmune disease. Rheumatoid arthritis occurs more frequently in women 30-60 years of age, and is estimated to affect around 700,000 individuals in Japan. Examples of the pathology of rheumatoid arthritis include synovial angiogenesis, infiltration of inflammatory cells, synovial cell proliferation, and destruction of cartilage and bone. In addition, arthritis caused by rheumatoid arthritis repeatedly improves and worsens as it progresses, gradually destroying cartilage and bone, and impeding the daily activities and reducing the quality of life (QOL) of rheumatoid arthritis patients.

In order to provide patients with the best care in the clinical therapy of rheumatoid arthritis, rheumatoid arthritis pathology/disease activity in patients is evaluated by calculating a specific index. In such index evaluations, evaluation items such as self-reported patient pain, testing and measurement of joints (numbers of painful joints, tender joints, swollen joints, etc.), and blood tests (erythrocyte sedimentation rate, rheumatoid factor, CRP, etc.) are combined to score rheumatoid arthritis pathology/disease activity. The severity of disease in the patient is then evaluated on the basis of the index score. It has also been reported that urinary interleukin-1 receptor antagonists can be used as an index (patent document 1).

### PRIOR ART LITERATURE

### [Patent Literature]

[Patent document 1] Japanese Patent No. 3530239 US 2016/194709 A1 (NAGY LÁSZLÓ [HU] ET AL) discloses a method to determine if a patient would respond to an anti-TNFalpha inhibitor treatment which mentions MS4A4A as one of the genes of which the expression can be used together with others (see claim 1, par 12). These genes were selected to predict the response of rheumatoid arthritis patients prior to starting treatment.

### SUMMARY OF THE INVENTION

### PROBLEM TO BE SOLVED BY THE INVENTION

Various types of rheumatoid arthritis therapeutic agents for administration to rheumatoid arthritis patients have been developed, and multiple variables such as strength of effects, administration method, time until effects manifest, risk of adverse reactions, severity of disease, disease activity, time since onset, complications, and therapy history are taken into account to select the rheumatoid arthritis therapeutic agent that it is believed will be most beneficial for that patient. However, there are individual differences in the effects of rheumatoid arthritis therapeutic agents, and drugs that are effective in some cases and patients are often ineffective in others. It is currently considered impossible to distinguish between responders and non-responders before drug administration.

Moreover, rheumatoid arthritis therapeutic agents are slow-acting, with many needing two to three months to manifest effects, and, in clinical settings, the therapeutic strategy of continuing administration of a given rheumatoid arthritis therapeutic agent for a minimum of three months once begun, and only changing to another therapeutic agent if no effects are seen after three months. It is therefore a reality that some patients are forced to continue with a completely ineffective therapy for several months.

Moreover, evaluations of rheumatoid arthritis pathology/disease activity using an activity index include self-reported patient pain, as discussed above, and thus require subjective judgment on the part of the patient. However, patient self-evaluations of pain can vary by individual or observer, making it difficult to objectively evaluate such activity. It is also difficult to comprehensively evaluate patient pathology/disease solely through currently utilized blood markers.

There is therefore a demand for the discovery of a more convenient and reproducible disease marker and the development of a method of directly assessing disease severity in order to diagnose rheumatoid arthritis and estimate therapy prognosis, or predict the efficacy of a rheumatoid arthritis therapeutic agent.

### MEANS FOR SOLVING THE PROBLEM

The present invention was conceived in view of these circumstances, and has an object of discovering a convenient and highly reproducible biomarker that enables prediction of the efficacy of a rheumatoid arthritis therapeutic agent and evaluation of rheumatoid arthritis pathology/disease activity, and providing an effective method for the same purposes.

As a result of focusing on MS4A4A gene expression levels in rheumatoid arthritis patients in order to solve this problem, the inventors observed that MS4A4A gene expression levels vary proportionally to the severity of rheumatoid arthritis pathological activity. Thus, as the result of diligent research into MS4A4A gene expression levels, the inventors discovered that the MS4A4A gene in blood sampled from subjects is capable of being a gene marker for predicting the efficacy of rheumatoid arthritis therapeutic agents, as well as a marker for evaluating rheumatoid arthritis pathology/disease activity, and discovered that, by measuring MS4A4A gene expression level, it is possible to predict the efficacy of a rheumatoid arthritis therapeutic agent and evaluate rheumatoid arthritis pathology/disease activity.

Specifically, a first main aspect of the present invention provides a method for predicting the efficacy of a rheumatoid arthritis therapeutic agent upon a rheumatoid arthritis patient, the method comprising steps of: measuring MS4A4A expression level in peripheral blood taken from the patient before starting administration of the rheumatoid arthritis therapeutic agent; and measuring MS4A4A expression level in peripheral blood taken from the patient after a first period of administering the rheumatoid arthritis therapeutic agent; wherein a decrease in MS4A4A expression level after the first period indicates a decrease in numerical value of an index associated with rheumatoid arthritis in the patient after a second period of administering the therapeutic agent.

This makes it possible to predict the efficacy of a rheumatoid arthritis therapeutic agent administered to a rheumatoid arthritis patient in a minimally invasive and convenient manner, without any particular burden upon the patient, simply by taking a blood sample. This also makes it possible to predict a numerical value for an index associated with rheumatoid arthritis in the patient after the second period simply by measuring MS4A4A expression level before starting administration of the rheumatoid arthritis therapeutic agent and after the first period, thus allowing the efficacy of the rheumatoid arthritis therapeutic agent to be evaluated without needing to continue administering the agent until after the second period.

The method according to the present invention is useful in that it is capable of predicting the future efficacy of a rheumatoid arthritis therapeutic agent without the need for subjective judgment of pain, etc., on the part of the patient, and thus enables objective prediction of the efficacy thereof. It is also possible to estimate a numerical value for an index associated with rheumatoid arthritis by measuring MS4A4A expression level in the blood, thus allowing for a simple, reproducible test that is more reliable than previous methods including patient self-assessments.

One embodiment of the present invention is the aforementioned method, wherein the first period is the period from administration of the therapeutic agent to four weeks thereafter, and the second period is the period from 12 weeks to 54 weeks after starting administration of the therapeutic agent.

Another embodiment of the present invention is the aforementioned method, wherein the therapeutic agent is selected from the group consisting of tocilizumab, methotrexate, and tumor necrosis factor inhibitors.

Another embodiment of the present invention is the aforementioned method, wherein the index is preferably selected from the group consisting of DAS28-CRP, DAS28-ESR, DAS44, European League Against Rheumatism (EULAR) response criteria, CDAI, SDAI, American College of Rheumatology (ACR) response criteria, and CRP level.

A second main aspect of the present invention provides a method for estimating a numerical value for an index associated with rheumatoid arthritis in a subject, the method comprising steps of: measuring MS4A4A expression level in peripheral blood taken from the subject; analyzing the measured expression level using a pre-prepared gene expression profile associated with the index; and estimating a numerical value for the index in the patient on the basis of the results of the analysis; the analyzing step being performed by comparing the measured MS4A4A gene expression level with a gene expression profile indicating the correlation between MS4A4A expression level and the index, the profile being created on the basis of MS4A4A gene expression levels in peripheral blood obtained from rheumatoid arthritis patients.

One embodiment of the present invention is the method according to the second main aspect of the present invention, wherein the index is selected from the group consisting of DAS28-CRP, DAS28-ESR, DAS44, EULAR response criteria, CDAI, SDAI, ACR response criteria, and CRP level; in this case, the index preferably indicates rheumatoid arthritis disease, or assesses the therapeutic effects of the rheumatoid arthritis therapeutic agent.

Another embodiment of the present invention is the aforementioned method, wherein a positive correlation in the gene expression profile and a high level of expression of the MS4A4A gene in peripheral blood taken from the subject indicate a high level of disease activity.

Another embodiment of the present invention is the aforementioned method, wherein the subject is affected by rheumatoid arthritis; preferably, in this case, the measuring step involves measuring MS4A4A expression level in peripheral blood taken before and after starting administration of the rheumatoid arthritis therapeutic agent to the subject, and the MS4A4A gene expression levels in the peripheral blood before and after starting administration are analyzed using the gene expression profile to assess the therapeutic effects of the therapeutic agent.

A third main aspect of the present invention provides an MS4A4A biomarker from peripheral blood for estimating a numerical value for an index associated with rheumatoid arthritis in a rheumatoid arthritis patient or a subject, the biomarker being used in the method described above.

Other features and salient effects of the present invention apart from those described above will become apparent to a person skilled in the art through reference to the following figures and embodiments of the invention.

### BRIEF DESCRIPTION OF THE DRAWINGS

[FIG. 1] FIG. 1 is a scatter plot showing results of an analysis of the correlation between MS4A4A expression level in a microarray and DAS28-ESR in the present invention.
[FIG. 2] FIG. 2 is a scatter plot showing results of an analysis of the correlation between MS4A4A expression level in PCR and DAS28-ESR in the present invention.
[FIG. 3] FIG. 3 is a graph of an analysis of the expression of the MS4A4A gene in healthy individuals, rheumatoid arthritis patients, and other diseases in the present invention.
[FIG. 4] FIG. 4 presents results showing variation in CDAI correlated with MS4A4A expression level in the present invention before and after starting administration of tocilizumab.
[FIG. 5] FIG. 5 presents results showing variation in DAS28-CRP, DAS28-ESR, ACR response criteria, EULAR-CRP, and EULAR-ESR correlated with MS4A4A expression level in the present invention before and after starting administration of tocilizumab.
[FIG. 6] FIG. 6 presents results showing variation in CDAI correlated with MS4A4A expression level in the present invention before and after starting administration of methotrexate.
[FIG. 7] FIG. 7 presents results showing variation in CDAI correlated with MS4A4A expression level in the present invention before and after starting administration of a tumor necrosis factor inhibitor.

### BESET MODE FOR EMBODYING THE INVENTION

An embodiment and examples of the present invention will be described below with reference to the figures.

As described above, a method for predicting the efficacy of a rheumatoid arthritis therapeutic agent upon a rheumatoid arthritis patient according to this embodiment comprises steps of: measuring MS4A4A expression level in peripheral blood taken from the patient before starting administration of the rheumatoid arthritis therapeutic agent; and measuring MS4A4A expression level in peripheral blood taken from the patient after a first period of administering the rheumatoid arthritis therapeutic agent; wherein a decrease in MS4A4A expression level after the first period indicates a decrease in numerical value of an index associated with rheumatoid arthritis in the patient after a second period of administering the therapeutic agent.

In one embodiment of the present invention, MS4A4A expression level varies in direct proportion to a rheumatoid arthritis activity index, and particularly reflects changes in numerical value for a rheumatoid arthritis activity index, as described in the examples hereinafter. Decreases in MS4A4A expression level after a first period of administering of a rheumatoid arthritis therapeutic agent indicate decreases in score for an index associated with rheumatoid arthritis after a second period of administering the therapeutic agent. As used herein, "gene marker" is used in substantially the same sense as "gene", and refers to a gene-related substance that serves as an index for evaluating the state or effects of a given object, and, in this case, correlates with the expression level of a gene. Examples include a gene itself, mRNA transcribed therefrom, a peptide translated therefrom, and a protein, which is the end product of gene expression. Unless stated otherwise, the "expression level" of a gene or protein refers to a standardized expression level of said gene or protein, and also encompasses the expression level of said gene in transcription (in the case of transcription products) or translation (in the case of polypeptides, proteins, etc.).

In this embodiment, "index associated with rheumatoid arthritis" includes, for example, DAS28-CRP, DAS28-ESR, DAS44, EULAR response criteria ( EULAR-CRP, EULAR-ESR), CDAI, SDAI, ACR response criteria, and CRP level, but may be any index that is capable of expressing the severity or activity of rheumatoid arthritis disease or pathology as a numerical value or score.

DAS (disease activity score) is an evaluation method recommended by EULAR (European League Against Rheumatism). DAS28 is a version of DAS narrowed down to 28 joints for ease of use in everyday medical care. In DAS28, four items, namely, (1) number of tender joints from 28 joints, (2) number of swollen joints from 28 joints, (3) one-hour erythrocyte sedimentation rate, or CRP (mg/dl), and (4) global health rating are assessed and converted to a numerical value according to a predetermined formula to calculate an absolute value for disease activity. DAS28-ESR is when erythrocyte sedimentation rate is used, and DAS28-CRP is when CRP is used. In the examples of the present invention, both DAS28-ESR and DAS28-CRP can be used for DAS28. DAS44 is a version in which 44 joints are assessed. In one embodiment of the present invention, DAS28-CRP and DAS28-ESR vary proportionally to changes in MS4A4A expression level, and the reference value for MS4A4A expression level (reference expression level) corresponding to a given DAS28-CRP or DAS28-ESR score can vary according to experimental conditions, etc., and is not limited to a specific numerical value. In DAS28-ESR, a score higher than 5.1 (4.1) is categorized as high disease activity, 3.2 (2.7) ≤ DAS28-ESR ≤ 5.1 (4.1) as moderate disease activity, 2.6 (2.3) < DAS28-ESR ≤ 3.2 (2.7) as low disease activity, and 2.6 or less as remission (numbers in parentheses are for DAS28-CRP). In the present invention, "remission" refers to a state of temporary or persistent alleviation of disease symptoms, or of no apparent symptoms.

In one embodiment of the present invention, EULAR response criteria(EULAR-CRP, EULAR-ESR) can also be used as the index associated with rheumatoid arthritis; the EULAR response criteria, for example, are based on DAS28. Two DAS28 values from before and after therapy (before and after drug administration) are combined to rate therapeutic effects according to three levels, good response, moderate response, and no response. CRP is used in the case of EULAR-CRP, and ESR in the case of EULAR-ESR. In one embodiment of the present invention, therapeutic effects in the subject can be rated according to the abovementioned three levels in proportion to change in MS4A4A expression level. In this embodiment, EULAR response criteria vary proportionally to change in MS4A4A expression level, and the reference value for change in MS4A4A expression level (reference expression level) corresponding to a given EULAR response criteria can vary according to experimental conditions, etc., and is not limited to a specific numerical value. For example, if a change in MS4A4A expression level corresponding to moderate response has been established, the gene expression profile can be analyzed to establish changes in expression level corresponding to good response or no response. In the present invention, a "decrease in numerical value for an index associated with rheumatoid arthritis" when EULAR response criteria or the like is used signifies an improvement in therapeutic effects, such as when previous no response becomes moderate response, or when previous moderate response becomes good response.

In one embodiment of the present invention, SDAI (Simple disease activity index) can be used as the index associated with rheumatoid arthritis. SDAI was developed as an evaluation that can easily calculated in clinical settings in response to the complexity of the calculation formula used in DAS, which cannot easily be calculated in clinical settings. SDAI evaluates the same joints as DAS28, and is calculated from the number of painful joints, the number of swollen joints, patient global assessment, and physician global assessment. In SDAI, a score of 26 or higher is categorized as high disease activity, 11 < SDAI ≤ 26 as moderate disease activity, 3.3 < SDAI ≤ 11 as low disease activity, and 3.3 or less as remission. In one embodiment of the present invention, SDAI score varies proportionally to changes in MS4A4A expression level, and the reference value for MS4A4A expression level (reference expression level) corresponding to a given SDAI score can vary according to experimental conditions, etc., and is not limited to a specific numerical value. For example, if an MS4A4A expression level corresponding to a specific SDAI score indicating moderate disease activity has been established, the gene expression profile can be analyzed to establish expression levels corresponding to specific scores indicating high disease activity and low disease activity as well.

In one embodiment of the present invention, CDAI (Clinical disease activity index) can be used as the index associated with rheumatoid arthritis. CDAI is SDAI without CRP. In CDAI, a score of 22 or higher is categorized as high disease activity, 10 < CDAI ≤ 22 as moderate disease activity, 2.8 < CDAI ≤ 10 as low disease activity, and 2.8 or less as remission. In this embodiment, CDAI score varies proportionally to changes in MS4A4A expression level, and the reference value for MS4A4A expression level (reference expression level) corresponding to a given CDAI score can vary according to experimental conditions, etc., and is not limited to a specific numerical value. For example, if an MS4A4A expression level corresponding to a specific CDAI score indicating moderate disease activity has been established, the gene expression profile can be analyzed to establish expression levels corresponding to specific scores indicating high disease activity and low disease activity as well.

In one embodiment of the present invention, ACR response criteria can be used as the index associated with rheumatoid arthritis. ACR response criteria refers to a set of guidelines for categorizing the pathological severity of rheumatoid arthritis established by the American College of Rheumatology. The ACR response criteria consist of seven measures constituting the ACR core set. The ACR core set consists of seven measures: (1) number of painful joints, (2) number of swollen joints, (3) patient disease assessment, (4) patient global assessment of disease activity, (5) physician global assessment of disease activity, (6) patient physical function assessment, and (7) acute-phase reactant. As an example, in ACR response criteria, 20% or better improvement in both (1) and (2) after therapy compared to before therapy (after drug administration compared to before drug administration) and 20% or better improvement in three out of the five measures (3) through (7) is assessed as "20% improvement per ACR standards (ACR20)". Similarly, ACR50 and ACR70 are used to indicate 50% and 70% improvements, respectively. As used herein, "ACR" without further qualification refers to "ACR response criteria", unless stated otherwise. In this embodiment, ACR varies proportionally to change in MS4A4A expression level, and the reference value for change in MS4A4A expression level (reference expression level) corresponding to a given ACR can vary according to experimental conditions, etc., and is not limited to a specific numerical value. For example, if a change in MS4A4A expression level corresponding to ACR50 has been established, the gene expression profile can be analyzed to establish expression levels corresponding to ACR20 or ACR70.

In one embodiment of the present invention, CRP level can be used as the index associated with rheumatoid arthritis. A normal CRP level is 0.3 mg/dl. In this embodiment, CRP level varies proportionally to changes in MS4A4A expression level, and the reference value for MS4A4A expression level (reference expression level) corresponding to a normal serum CRP value can vary according to experimental conditions, etc., and is not limited to a specific numerical value.

In one embodiment of the present invention, the method for predicting the efficacy of a rheumatoid arthritis therapeutic agent upon a rheumatoid arthritis patient is performed by measuring MS4A4A expression levels in blood taken from a patient before and after receiving the rheumatoid arthritis therapeutic agent. An example of subject sample is blood sampled from the subject; the subject sample may be whole blood, peripheral blood, or cell components isolated from the blood. There is no particular limitation upon the method by which cell components are isolated from the blood, and various conventional methods, such as methods for isolating cell components from blood for use in clinical test samples, may be followed. For example, cells obtained by centrifuging sampled blood in a test tube or the like at room temperature under specific conditions can be used as a test sample.

This embodiment of the method for predicting the efficacy of a rheumatoid arthritis therapeutic agent upon a rheumatoid arthritis patient according to the present invention is advantageous in that, because testing can conveniently be performed upon samples of blood taken from a patient, it is easier to collect samples than methods in which synovial fluid is sampled for testing, and test samples used in ordinary clinical tests can be used. Synovial fluid is collected via invasive methods, and thus places a high burden on the patient in terms of pain, etc., and also presents the risk of complications such as hemorrhaging or infection; thus, synovial fluid is not particularly preferable for routine test methods. Meanwhile, using blood samples to measure MS4A4A expression level, as in the present invention, eliminates these problems, allows a numerical value for an index associated with rheumatoid arthritis in a subject to be conveniently estimated, and thus makes it possible to predict the efficacy of a rheumatoid arthritis therapeutic agent. As used herein, "MS4A4A level" refers to MS4A4A expression level, unless specifically noted otherwise.

In one embodiment of the method for predicting the efficacy of a rheumatoid arthritis therapeutic agent upon a patient according to the present invention, the MS4A4A gene in the sampled blood is a maker of a rheumatoid arthritis activity index. A rheumatoid arthritis activity index or disease/pathology can be identified from measurement results for MS4A4A expression level obtained via the method of the present invention, and rheumatoid arthritis can be diagnosed or the efficacy of a rheumatoid arthritis therapeutic agent can be predicted therefrom.

In one embodiment of the present invention, the efficacy of a rheumatoid arthritis therapeutic agent upon a rheumatoid arthritis patient can be predicted using the method of the present invention by sampling blood from the rheumatoid arthritis patient before and after starting administration of the rheumatoid arthritis therapeutic agent, and measuring MS4A4A expression levels in the blood sampled before and after starting administration. This embodiment is characterized in that a pre-prepared gene expression profile correlated with an index associated with rheumatoid arthritis is subsequently used to compare the MS4A4A expression levels of the blood samples.

In this case, blood can be sampled from the rheumatoid arthritis patient after a first period as the blood sample taken after starting administration of the rheumatoid arthritis therapeutic agent. This "first period" can be a period of up to four weeks of administration of the therapeutic agent, or may be week 1, week 2, week 3, or week 4, or day 3, day 5, day 10, day 12, day 15, day 20, day 25, etc., of administering the therapeutic agent, depending on the type of therapeutic agent, severity of disease, administration method, time until effects manifest, disease activity, time following onset, complications, therapy history, and so forth.

In one embodiment of the present invention, "after a second period of administering the therapeutic agent" can be after a period of 12-54 weeks of administering the therapeutic agent. This second period can also be established according to type of therapeutic agent, severity of disease, administration method, time until effects manifest, disease activity, time following onset, complications, therapy history, and so forth, and can be, for example, week 12, week 14, week 16, week 22, week 24, week 30, week 32, week 38, week 40, week 46, week 48, week 52, week 54, etc.

In one embodiment of the present invention, the results of predicting the efficacy of a rheumatoid arthritis therapeutic agent as described above reveal a close association between change in MS4A4A expression level before and after starting administration the therapeutic agent and future actual evaluation of efficacy. For example, it is possible to compare the MS4A4A expression level measured before starting administration (week 0) and the MS4A4A expression level measured on the fourth week of administration of the therapeutic agent, and, if the latter is lower, predict that the numerical value for the index associated with rheumatoid arthritis will have decreased, or the assessment of therapeutic effects will have improved, on, for example, week 46, week 48, week 52, or week 54 (see the examples described below). In other words, not only does the relationship between MS4A4A expression level measurement results and index associated with rheumatoid arthritis and the evaluation of the index associated with rheumatoid arthritis closely reflect the degree of therapeutic effects, but the degree of change in MS4A4A expression level before and after therapy is also related to the future efficacy of the rheumatoid arthritis therapeutic agent. This can be extremely profitable clinical information in planning therapies using rheumatoid arthritis therapeutic agents, many of which require two to three months of administration before showing effects, as the decision whether or not to continue administering the drug can be made within four weeks after starting therapy, at a comparatively early stage.

In the present invention, there is no particular limitation upon the "rheumatoid arthritis therapeutic agent", and currently known therapeutic agents as well as any agents developed in the future are encompassed thereby. Examples of known rheumatoid arthritis therapeutic agents include conventional synthetic disease-modifying antirheumatic drugs such as methotrexate (MTX), biological disease-modifying antirheumatic drugs (synonymous with biologics), molecular-targeted synthetic disease-modifying antirheumatic drugs such as Janus kinase (JAK) inhibitors, non-steroidal anti-inflammatory drugs (anti-inflammatory analgesics), steroids, immunosuppressants, and the like. Examples of biologics include chimeric anti-TNF-α antibody preparations, soluble TNF receptors, fully human anti-TNF-α antibody preparations, and anti-Interleukin-6 (IL-6) receptor antibody preparations. Examples of non-steroidal anti-inflammatory drugs include prostaglandin production inhibitors, Specific non-limiting examples of the rheumatoid arthritis therapeutic agent in the present invention include MTX, the tumor necrosis factor inhibitor (TNFi) infliximab (IFX), etanercept (ETN), adalimumab (ADA), and the anti-IL-6 receptor antibody preparation tocilizumab (TCZ).

In one embodiment of the present invention, it is possible to estimate a numerical value for an index associated with rheumatoid arthritis in a subject; this method comprises steps of: measuring MS4A4A expression level in peripheral blood taken from the subject; analyzing the measured expression level using a pre-prepared gene expression profile associated with the index; and estimating a numerical value for the index in the patient on the basis of the results of the analysis; the analyzing step being performed by comparing the measured MS4A4A gene expression level with a gene expression profile indicating the correlation between MS4A4A expression level and the index, the profile being created on the basis of MS4A4A gene expression levels in peripheral blood obtained from rheumatoid arthritis patients.

In one embodiment of the present invention, a numerical value for the index associated with rheumatoid arthritis can be estimated through comparative analysis of subject MS4A4A expression level and a gene expression profile prepared in advance from MS4A4A expression levels in various diseased and healthy sample groups, including rheumatoid arthritis patients and healthy individuals, and groups receiving or not receiving various types of rheumatoid arthritis therapeutic agents. Alternatively, a numerical value can be estimated for the index associated with rheumatoid arthritis through comparative analysis of gene expression profiles for the same subject (including healthy individuals and rheumatoid arthritis patients) before and after receiving the rheumatoid arthritis therapeutic agent to estimate a numerical value for the index associated with rheumatoid arthritis in the subject. In this case, the various diseased and healthy sample groups can be sampled according to factors such as sex, age, type of therapeutic agent, time since receiving therapeutic agent. The cumulative sample size of the pre-prepared gene expression profile is preferably as large as possible, as this will increase the precision of the identification method of the present invention. In the present invention, this cumulative sample data and the pre-prepared gene expression profile can be configured to be storable in any desired database. In other words, the present invention can also provide a database of such data and an analysis device for reading and executing programs, etc., necessary for said data and comparative analysis. Using such an analysis device, it is possible to conveniently estimate a value for an index associated with rheumatoid arthritis in a subject whenever desired simply by retrieving the cumulative data from the database and comparing with the data measured for the subject.

In this embodiment, estimation/analysis of the numerical value for the index associated with rheumatoid arthritis in the subject can be performed by comparing the measured MS4A4A expression level with a gene expression profile created from MS4A4A expression levels in various diseased and healthy states, and any period that is clinically significant for evaluating rheumatoid arthritis disease state can be set for the period after starting administration the therapeutic agent.

In this embodiment, the gene expression profile described above preferably indicates a positive correlation between MS4A4A expression level and a given index associated with rheumatoid arthritis. Thus, for example, if MS4A4A expression level in blood taken from a subject is higher than a reference expression level, said index will indicate disease activity that is higher than a reference value.

In one embodiment of the present invention, any analysis means used in fields such as molecular biology or bioinformatics, such as cluster analysis and various algorithms, can be used to create/generate the gene expression profile and analyze data.

In one embodiment of the present invention, the abovementioned MS4A4A gene can be used as a biomarker for estimating a numerical value for an index associated with rheumatoid arthritis in a rheumatoid arthritis patient or a subject. MS4A4A, also referred to as CD20-like 1, belongs to the MS4A family of four-transmembrane proteins including CD20 (MS4A1) and FceRIb (MS4A2). 18 proteins in the family have been identified, each of which exhibits around 20-32% homology.

### (Experimental methods)

Experimental methods and materials used to prove the effects of the gene marker according to an embodiment of the present invention, as well as definitions for the same, will be described below. While the experimental methods described below are used in this embodiment, similar results can be obtained using other methods.

### (Gene quantification)

If the gene marker in this embodiment is a gene or a gene transcription product (mRNA), measurement (quantification) of gene expression level can be performed by measuring mRNA levels using various types of molecular biological methods, such as microarray, northern blot, dot blot, quantitative RT-PCR (quantitative reverse transcription polymerase chain reaction; real-time RT-PCR), next-generation sequencing, and digital PCR.

While there is no particular limitation upon the primer used in PCR methods as long as it is capable of specifically detecting a gene or gene marker, a 12-26 base oligonucleotide is preferable. The base sequence thereof is determined on the basis of sequence information for various human genes. A primer having the determined sequence can then be prepared using a DNA synthesizer.

Meanwhile, if the gene marker is a translation product (polypeptide) or end product (protein) of a gene, the expression level of the gene marker can be measured, for example, via western blot using polyclonal or monoclonal antibodies specific to the gene marker, ELISA, or the like, without limitation thereto; various methods such as RIA (radioimmunoassay), EIA (enzyme immunoassay) or the like can also be used.

"Gene" refers to genetic information encoded in base sequences such as RNA and DNA. Orthologous genes preserved between species such as human, mouse, and rat are also included. The gene may not only code a protein, but also function as RNA or DNA. Generally, the gene will code a protein conforming with the base sequence thereof, but may also code a protein having a biological function comparable to that of said protein (e.g., related genes such as homologs and splicing variants; mutants; and derivatives). For example, the gene may code a protein having a base sequence that differs slightly from the base sequence indicated by the genetic information, and hybridizes with a sequence complementary to the sequence indicated by the genetic information.

"RNA" is a concept encompassing not only single-stranded RNA, but also single-stranded RNA having sequences complementary thereto, and double-stranded RNA constituted by these two. The concept also encompasses total RNA, mRNA and rRNA.

"Expression level" is a concept encompassing not only directly measured values for gene expression level, but also values converted via specific calculations or statistical methods. "Gene expression level", "expression signal", "gene expression signal", "expression signal value", "gene expression signal value", "gene expression data", "expression data", and the like are synonymous in that these terms refer to values that reflect the expression of individual genes.

"Gene expression" refers to *in vivo* gene expression as manifested by gene expression level, and includes both single-gene expression levels and multiple-gene expression levels. "Expression" is synonymous in that the term refers to *in vivo* gene expression.

### EXAMPLES

The present invention will be described in greater detail below with reference to examples, but is not limited thereto.

### (Marker gene searching/selection)

A method of searching for and selecting a marker gene according to the present invention will be described below.

The inventors attempted to extract a novel marker gene that reflects rheumatoid arthritis disease activity from blood sample microarray data for 611 rheumatoid arthritis patients having received care at the Department of Rheumatology and Immunology at Saitama Medical Center and the Department of Rheumatology at Keio University Hospital, who had received and signed a consent form to participate in research.

RNA was extracted from subject blood using the PAXgene Blood RNA System (manufactured by QIAGEN). Extracted RNA yield was measured using a NanoDrop 1000 (manufactured by Thermo Scientific), and was confirmed to be degradation-free using a 2100 Bioanalyzer (manufactured by Agilent Technologies). Next, cRNA was amplified from 250 ng RNA via an *in vitro* transcription reaction using an Agilent Technologies Low RNA Input Linear Amp Kit PLUS, One-Color, and simultaneously fluorescently labeled (Cy3 labeled). Next, the fluorescently labeled cRNA was hybridized for 17 hours at 65°C using an Agilent Technologies Whole Human Genome Microarray 4×44k. After washing with Agilent Technologies Gene Expression Wash Buffer, a fluorescent image was scanned using an Agilent Scanner (manufactured by Agilent Technologies), and the signal strength of spots in the fluorescent image was quantified using Agilent Technologies' Feature Extraction image quantification software.

The quantified data was normalized via quantile normalization, and experimental batch adjustment processing was performed according to the ComBat method (Biostatistics, Jan 2007; 8(1): 118-27). Spearman's correlation, the Pearson product-moment correlation coefficient, and a test for no correlation p value were calculated for the normalized value (Log 2 scale) of the probe and the DAS28-ESR of the subject, and a gene correlated with disease activity was extracted.

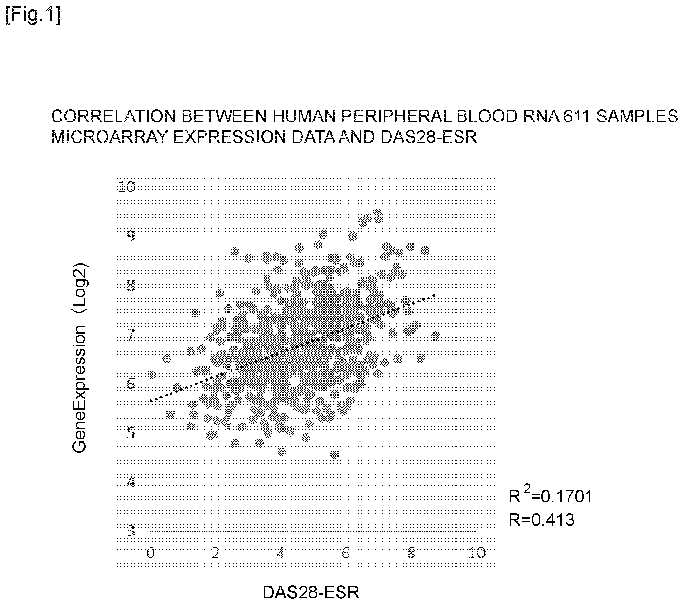

As a result, a probe detecting MS4A4A transcripts was extracted as a probe significantly correlated with DAS28-ESR. High expression of said gene in peripheral blood is induced as rheumatoid arthritis disease activity increases (FIG. 1).

### (PCR analysis of MS4A4A gene)

Next, real-time PCR analysis was performed to confirm the reproducibility of the correlation between peripheral blood MS4A4A expression in rheumatoid arthritis patients and rheumatoid arthritis activity.

Real-time PCR reactions were performed on 48 cases of rheumatoid arthritis patient peripheral blood using an Applied Biosystems Power SYBR Green RNA-to-CT 1-Step Kit. 20 ng RNA was used in the experiment. Adjustment was performed using the comparative Ct method which calculates -ΔCt value (MS4A4A transcript - internal standard gene transcript).

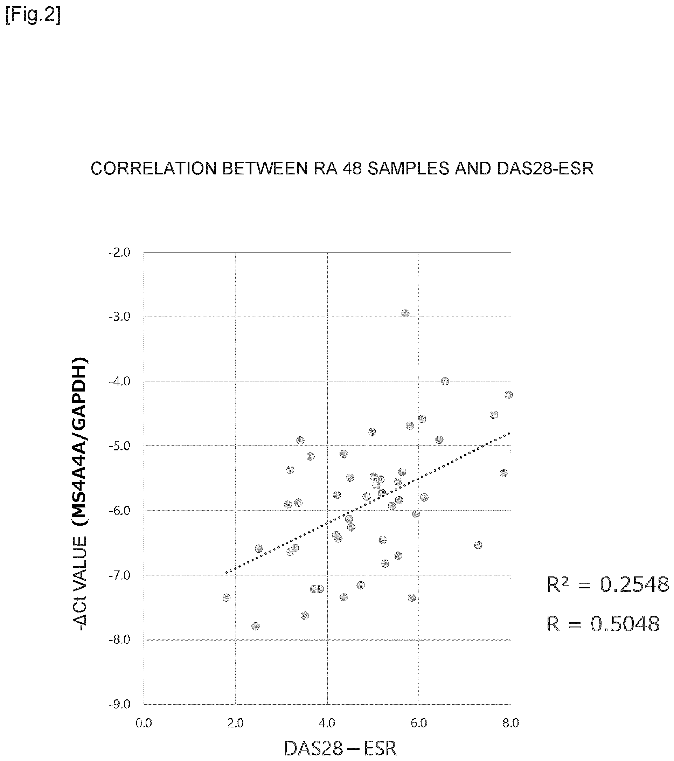

FIG. 2 is a scatter plot illustrating the correlation between MS4A4A -ΔCt values (difference from control) obtained via real-time PCR data and DAS28-ESR. Correlation analysis results for MS4A4A and various clinical test measures are shown in Table 1.

**[TABLE 1]**

| **CLINICAL INFORMATION ASSOCIATED WITH MS4A4A** | | | | |
|---|---|---|---|---|
| **ANALYSIS USING qPCR DATA FROM 48 CASES OF SAMPLES FROM PATIENTS WITH UNTREATED RA** | | | | |
| | MS4A4A qPCR | | | |
| | Spearman | | Pearson | |
| Measure | Rho | p-value | R | p-value |
| FAM20A | 0.39752 | 0.005448357 | 0.43996 | 0.001753443 |
| MS4A4A | - | - | - | - |
| Age | 0.1 | 0.498561577 | 0.06 | 0.70026328 |
| Disease duration | -0.31 | 0.03122909 | -0.11 | 0.474833752 |
| TJC28 | 0.31 | 0.029863163 | 0.27 | 0.058951708 |
| SIC28 | 0.34 | 0.01883756 | 0.30 | 0.035659841 |
| TJC48 | 0.28 | 0.056078358 | 0.32 | 0.027894141 |
| SIC48 | 0.27 | 0.059954018 | 0.33 | 0.023110498 |
| P-VAS | 0.21 | 0.159399901 | 0.20 | 0.173735657 |
| GH | 0.24 | 0.105869417 | 0.22 | 0.131120134 |
| D-VAS | 0.38 | 0.007967027 | 0.35 | 0.013847768 |
| HAQ | 0.36 | 0.011993203 | 0.33 | 0.021232728 |
| CRP | 0.22 | 0.134508643 | 0.29 | 0.042536144 |
| ESR | 0.54 | 8.65E-05 | 0.44 | 0.001626684 |
| DAS28-CRP | 0.34 | 0.018822856 | 0.39 | 0.005566326 |
| DAS28-ESR | 0.48 | 0.000628545 | 0.50 | 0.000253229 |
| SDAI | 0.43 | 0.002495967 | 0.38 | 0.007668159 |
| CDAI | 0.39 | 0.005674049 | 0.35 | 0.015166721 |
| RBC | -0.42 | 0.003325492 | -0.44 | 0.001792775 |
| Hemoglobin | -0.38 | 0.007454322 | -0.39 | 0.005800049 |
| WBC | -0.08 | 0.580905455 | 0.05 | 0.748035074 |
| Neutrophils | 0.24 | 0.107284924 | 0.18 | 0.231768819 |
| Eosinophils | -0.20 | 0.171396466 | 0.07 | 0.637572301 |
| Basophils | -0.08 | 0.569015405 | -0.04 | 0.765351446 |
| Mononuclear cells | 0.27 | 0.068590897 | 0.29 | 0.047824691 |
| Lymphocytes | -0.29 | 0.047961832 | -0.27 | 0.067904877 |
| Platelets | 0.23 | 0.121638291 | 0.19 | 0.194812519 |
| AST | 0.00 | 0.993259118 | 0.10 | 0.515081944 |
| ALT | -0.07 | 0.616829388 | 0.12 | 0.433848762 |
| LDH | 0.24 | 0.102342238 | 0.36 | 0.01097707 |
| ALP | -0.04 | 0.782759384 | -0.08 | 0.617456921 |
| Total protein | 0.00 | 0.992516502 | 0.03 | 0.817773983 |
| Albumin | -0.42 | 0.003356313 | -0.39 | 0.006344914 |
| Total cholesterol | -0.25 | 0.119374141 | -0.19 | 0.234243705 |
| Serum creatinine | -0.24 | 0.102180031 | -0.29 | 0.041951909 |
| MMP-3 | 0.26 | 0.087852623 | 0.19 | 0.207056276 |
| Rheumatoid factor titer | 0.46 | 0.000902277 | 0.47 | 0.000858975 |
| Antinuclear antibodies | 0.19 | 0.206774341 | -0.10 | 0.501031755 |
| Anti-dsDNA antibodies | 0.14 | 0.399569309 | 0.16 | 0.325190578 |
| IgG | 0.25 | 0.083371069 | 0.32 | 0.025359595 |
| IgA | 0.19 | 0.199178314 | 0.22 | 0.136930494 |
| IgM | 0.12 | 0.41342309 | 0.05 | 0.759503333 |

From these results, significant positive correlations were observed between MS4A4A expression level and ESR, which is a DAS component, and SDAI, CDAI, and rheumatoid factor quantification value, which are indexes of rheumatoid arthritis disease activity. From this, it is believed that rheumatoid arthritis activity can be objectively and comprehensively surveyed by measuring peripheral blood MS4A4A expression level. The abovementioned results also make it clear that the correlation between peripheral blood MS4A4A expression in rheumatoid arthritis patients and rheumatoid arthritis activity is reproducible not only in microarray analysis, but also in real-time PCR.

### (Comparison with healthy individuals and other diseases)

Next, the difference in peripheral blood MS4A4A gene expression between healthy individuals and rheumatoid arthritis patients was analyzed using real-time PCR. MS4A4A expression levels in rheumatoid arthritis patients, healthy individuals, and patients of other diseases, specifically, vasculitis, adult-onset Still's disease, and systemic lupus erythematosus, were compared to investigate the potential of MS4A4A as a diagnostic marker for rheumatoid arthritis.

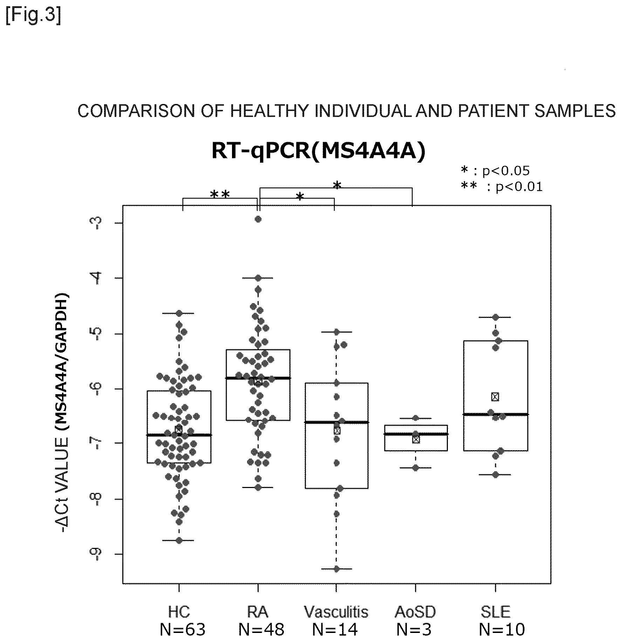

48 cases were analyzed as rheumatoid arthritis patient samples (RA). Peripheral blood samples (HC) from 63 healthy individuals who had visited a medical institution for health checkup, and had received and signed a consent form to participate in peripheral blood gene expression analysis research, were analyzed as healthy individual samples. Peripheral blood samples from 14 vasculitis patients, three adult-onset Still's disease (AoSD) patients, and 10 systemic lupus erythematosus (SLE) patients who had received and signed a consent form to participate in peripheral blood gene expression analysis research were used as disease control samples. The distribution of MS4A4A -ΔCt values (difference from control) in each sample group are shown in FIG. 3.

As a result of a Student's t-test, significant differences (p < 0.05 or 0.01) were observed between rheumatoid arthritis patients and healthy individuals, vasculitis patients, and adult-onset Still's disease patients. These results suggest potential for MS4A4A not only as a marker for evaluating rheumatoid arthritis activity, but also as a diagnostic marker for rheumatoid arthritis that enables differentiation from other diseases, including inflammatory disease.

### (Variation in MS4A4A expression caused by administration of rheumatoid arthritis therapeutic agent)

An investigation was performed into how MS4A4A expression varies in the same patient in the early stage (up to four weeks) after receiving a rheumatoid arthritis therapeutic agent, as well as the association between such variation and subsequent therapeutic effects.

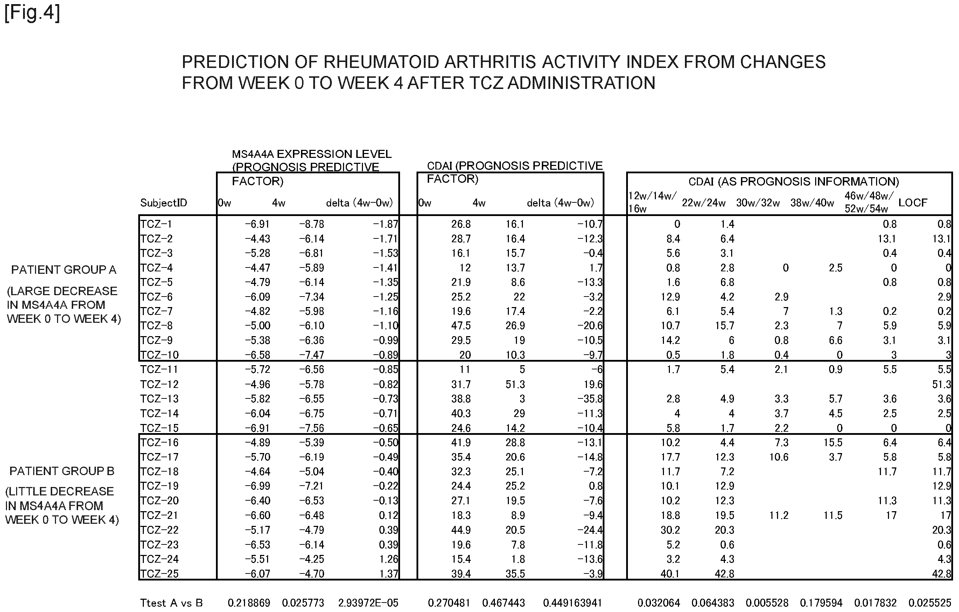

In FIG. 4, MS4A4A expression levels in 25 rheumatoid arthritis patients were measured before receiving TCZ (0w) and in week 4 of administration (4w), and the association between the difference therebetween and therapeutic effects (activity index: CDAI) from week 12 (12w) to week 54 (54w) of administration. Groups in which there was a major decrease in MS4A4A expression level from 0w to 4w had significantly lower CDAI values for 12w to 54w of administration, and better TCZ therapy response, than groups in which there was only a slight decrease. Meanwhile, no such trend was observed for the difference in CDAI values between 0w and 4w.

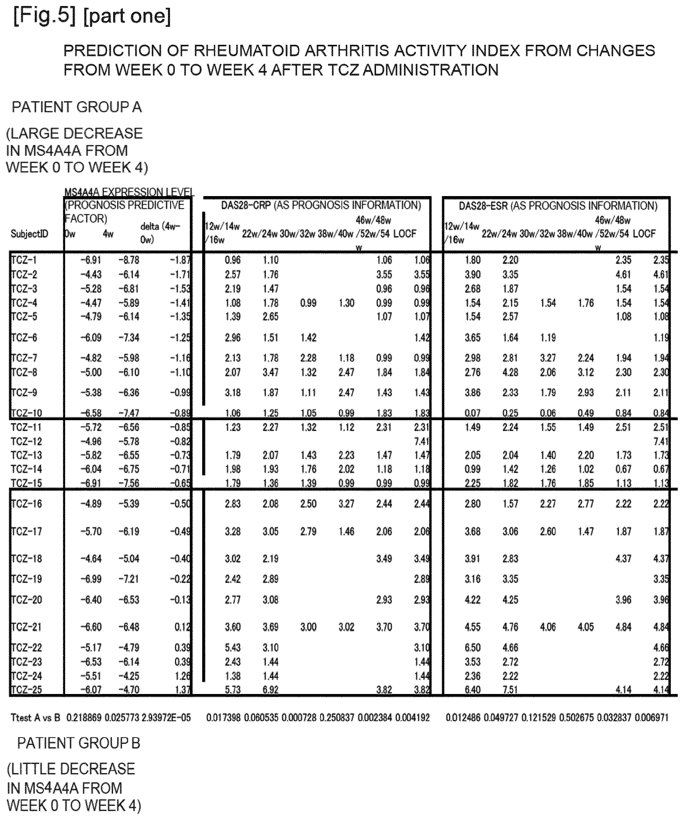

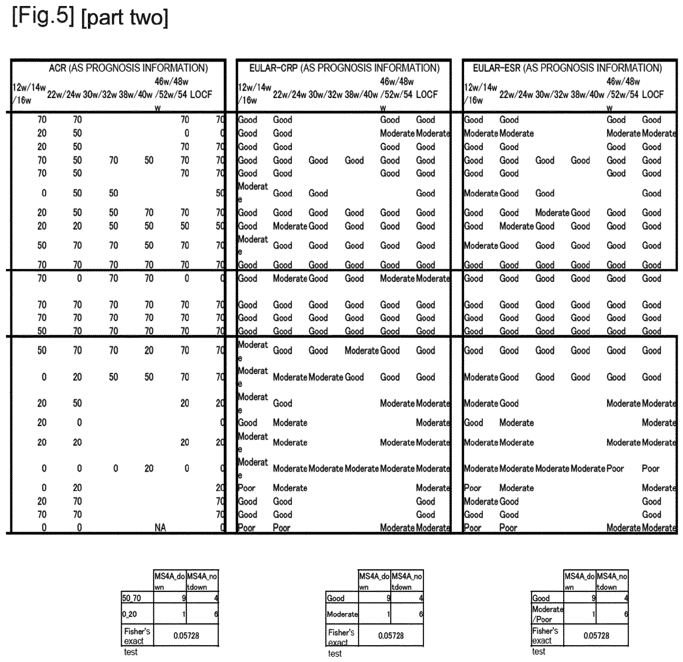

FIG. 5 is an evaluation of the other activity indexes DAS28-CRP, DAS28-ESR, ACR, EULAR-CRP, and EULAR-ESR, rather than CDAI, for the same 25 TCZ-receiving cases. Trends similar to those observed for CDAI were seen in all the other activity indexes, and groups in which there was a major decrease in MS4A4A expression level from 0w to 4w had good therapy response from 12w to 54w. The foregoing results reveal that therapy response from 12w of TCZ administration onward can be predicted from changes in MS4A4A expression level in the early stages after beginning TCZ administration.

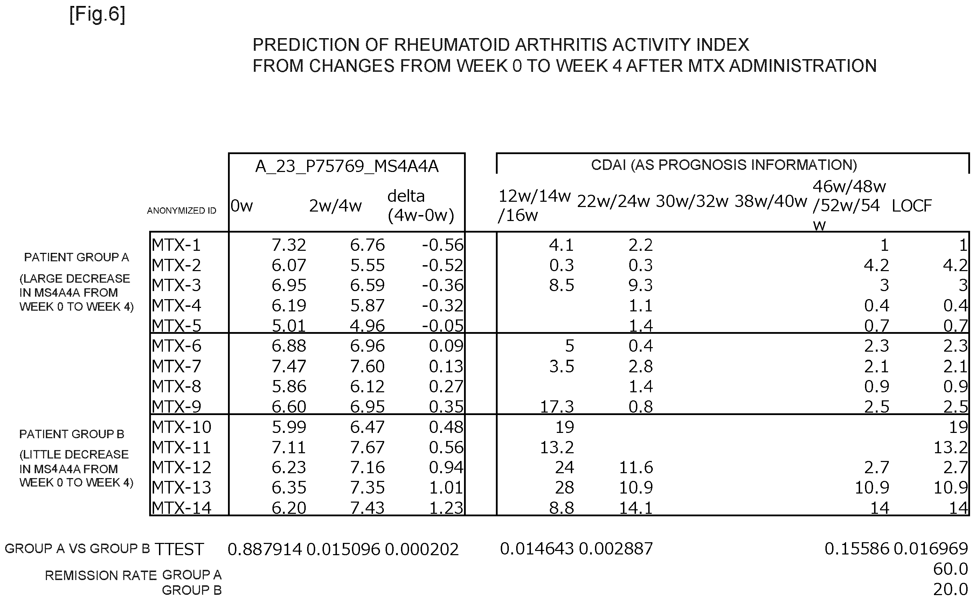

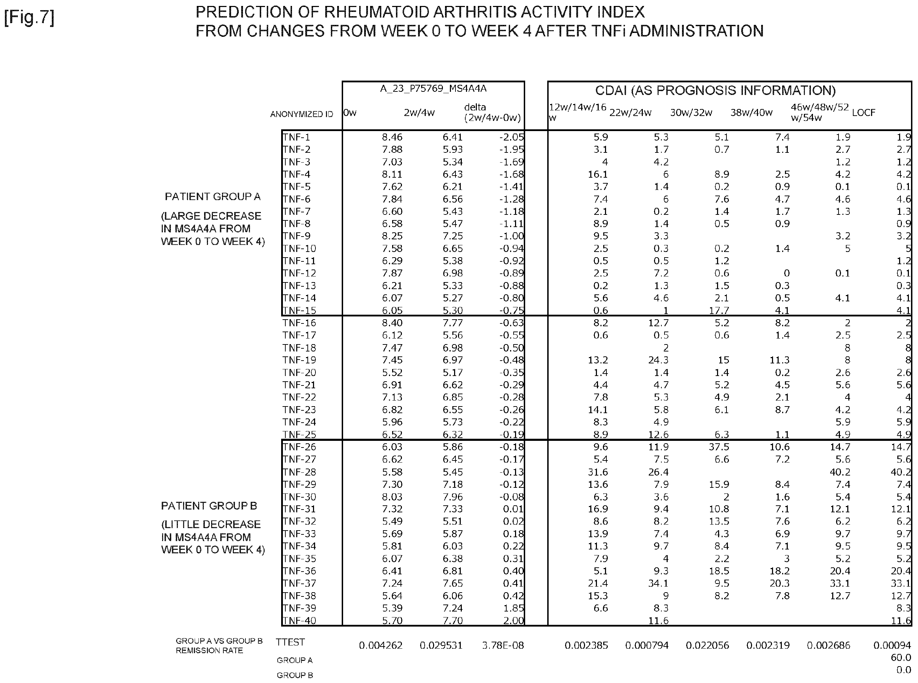

FIG. 6 and 7 are evaluations of rheumatoid arthritis patients who received MTX and TNFi therapy. Follow-up regarding therapeutic effects in patients in which there was a major decrease in MS4A4A expression level (five MTX cases, 15 TNFi cases) from 0w to 4w (2w in the case of TNFi) and patients in which there was no such decrease revealed a tendency toward better therapeutic outcome in the former.

The results described above show that peripheral blood MS4A4A expression level is a marker that reflects the future effects of a rheumatoid arthritis therapeutic agent upon a rheumatoid arthritis patient.

## Claims

1. A method for predicting the efficacy of a rheumatoid arthritis therapeutic agent upon a rheumatoid arthritis patient, the method comprising steps of:
measuring MS4A4A expression level in peripheral blood taken from the patient before starting administration of the rheumatoid arthritis therapeutic agent; and
measuring MS4A4A expression level in peripheral blood taken from the patient after a first period of administering the rheumatoid arthritis therapeutic agent;
wherein a decrease in MS4A4A expression level after the first period indicates a decrease in numerical value of an index associated with rheumatoid arthritis in the patient after a second period of administering the therapeutic agent.

2. The method according to claim 1, wherein the first period is a period of up to four weeks of administering the therapeutic agent.

3. The method according to claim 1, wherein the second period is a period of 12 weeks to 54 weeks of administering the therapeutic agent.

4. The method according to claim 1, wherein the therapeutic agent is selected from the group consisting of tocilizumab, methotrexate, and tumor necrosis factor inhibitors.

5. The method according to claim 1, wherein the index is selected from the group consisting of DAS28-CRP, DAS28-ESR, DAS44, EULAR response criteria, CDAI, SDAI, ACR response criteria, and CRP level.

6. A method for estimating a numerical value for an index of a disease activity associated with rheumatoid arthritis in a subject, the method comprising steps of:
measuring MS4A4A expression level in peripheral blood taken from the subject;
analyzing the measured expression level using a pre-prepared gene expression profile correlated with the index; and
estimating a numerical value for the index in the subject on the basis of the results of the analysis;
the analyzing step being performed by comparing the measured MS4A4A gene expression level with a gene expression profile indicating the correlation between MS4A4A expression level and the index, the profile being created on the basis of MS4A4A gene expression levels in peripheral blood obtained from rheumatoid arthritis patients and index of disease activity of the rheumatoid arthritis patient, wherein the index is selected from the group consisting of DAS28-CRP, DAS28-ESR, DAS44, EULAR response criteria, CDAI, SDAI, ACR response criteria, and CRP level.

7. The method according to claim 6, wherein the index indicates rheumatoid arthritis disease activity, or assesses the therapeutic effects of a rheumatoid arthritis therapeutic agent.

8. The method according to claim 6, wherein a positive correlation in the gene expression profile and a high level of expression of the MS4A4A gene in peripheral blood taken from the subject indicates a high level of disease activity.

9. The method according to claim 6, wherein the subject is affected by rheumatoid arthritis.

10. The method according to claim 9, wherein the measuring step involves measuring MS4A4A expression level in peripheral blood taken before and after starting administration of the rheumatoid arthritis therapeutic agent to the subject, and the MS4A4A gene expression levels in the peripheral blood before and after starting administration are analyzed using the gene expression profile to assess the therapeutic effects of the therapeutic agent.

11. The method according to claim 1, wherein the first period is a period of up to four weeks of administering the therapeutic agent and the second period is a period of 46 weeks to 54 weeks of administering the therapeutic agent.

12. The method according to claim 6, comprising a step of distinguishing the subject from rheumatoid arthritis patients and healthy individuals and a patient with a disease other than the rheumatoid arthritis, on the basis of the MS4A4A gene expression levels.

## Patentansprüche

1. Das Verfahren zum Vorhersagen der Wirksamkeit eines Therapeutikums für rheumatoide Arthritis bei einem Patienten mit rheumatoider Arthritis, wobei das Verfahren die folgenden Schritte umfasst:
Messen des MS4A4A-Expressionsniveaus in peripherem Blut, das dem Patienten vor Beginn der Verabreichung des Therapeutikums gegen rheumatoide Arthritis entnommen wurde; und
Messen des MS4A4A-Expressionsspiegels in peripherem Blut, das dem Patienten nach einer ersten Periode der Verabreichung des Therapeutikums für rheumatoide Arthritis entnommen wurde;
wobei eine Abnahme des MS4A4A-Expressionsniveaus nach der ersten Periode auf eine Abnahme des Zahlenwerts eines mit rheumatoider Arthritis assoziierten Index beim Patienten nach einer zweiten Periode der Verabreichung des therapeutischen Wirkstoffs hinweist.

2. Das Verfahren nach Anspruch 1, wobei der erste Zeitraum ein Zeitraum von bis zu vier Wochen umfasst, in dem der therapeutische Wirkstoff verabreicht wird.

3. Das Verfahren nach Anspruch 1, wobei die zweite Periode ein Zeitraum von 12 bis 54 Wochen ist, in dem der therapeutische Wirkstoff verabreicht wird.

4. Das Verfahren nach Anspruch 1, wobei der therapeutische Wirkstoff ausgewählt ist aus der Gruppe bestehend aus Tocilizumab, Methotrexat und Tumornekrosefaktor-Inhibitoren.

5. Das Verfahren nach Anspruch 1, wobei der Index aus der Gruppe ausgewählt ist, die aus DAS28-CRP, DAS28-ESR, DAS44, EULAR-Ansprechkriterien, CDAI, SDAI, ACR-Ansprechkriterien und CRP-Ebene besteht.

6. Das Verfahren zum Schätzen eines numerischen Werts für einen Index einer Krankheitsaktivität, die mit rheumatoider Arthritis in einem Subjekt assoziiert ist, wobei das Verfahren die folgenden Schritte umfasst:
Messen des MS4A4A-Expressionsniveaus in peripherem Blut, das dem Probanden entnommen wurde;
Analysieren des gemessenen Expressionsniveaus unter Verwendung eines vorbereiteten Genexpressionsprofils, das mit dem Index korreliert; und
Schätzen eines numerischen Wertes für den Index im Subjekt auf der Grundlage der Ergebnisse der Analyse;
Analyseschritt durch Vergleichen des gemessenen MS4A4A-Genexpressionsniveaus mit einem Genexpressionsprofil durchgeführt wird, das die Korrelation zwischen dem MS4A4A-Expressionsniveau und dem Index angibt, wobei das Profil auf der Grundlage der MS4A4A-Genexpressionsniveaus in peripherem Blut, das von Patienten mit rheumatoider Arthritis erhalten wurde, und des Index der Krankheitsaktivität des Patienten mit rheumatoider Arthritis erstellt wird, wobei der Index aus der Gruppe bestehend aus DAS28-CRP, DAS28-ESR, DAS44, EULAR-Ansprechkriterien, CDAI, SDAI, ACR-Ansprechkriterien und CRP-Niveau ausgewählt ist.

7. Das Verfahren nach Anspruch 6, wobei der Index die Aktivität der rheumatoiden Arthritis-Krankheit anzeigt oder die therapeutischen Wirkungen eines Therapeutikums für rheumatoide Arthritis bewertet.

8. Das nach Anspruch 6, wobei eine positive Korrelation im Genexpressionsprofil und eine hohe Expression des MS4A4A-Gens in peripherem Blut, das dem Patienten entnommen wurde, auf eine hohe Krankheitsaktivität hinweisen.

9. Das Verfahren nach Anspruch 6, wobei das Subjekt von rheumatoider Arthritis betroffen ist.

10. Das Verfahren nach Anspruch 9, wobei der Messschritt das Bestimmen des MS4A4A-Expressionsniveaus in peripherem Blut umfasst, das dem Subjekt vor und nach Beginn der Verabreichung des Therapeutikums gegen rheumatoide Arthritis entnommen wird, und wobei die MS4A4A-Genexpressionsniveaus im peripheren Blut vor und nach Beginn der Verabreichung unter Verwendung eines Genexpressionsprofils analysiert werden, um die therapeutischen Wirkungen des therapeutischen Wirkstoffs zu bewerten.

11. Das Verfahren nach Anspruch 1, wobei die erste Periode ein Zeitraum von bis zu vier Wochen ist, in dem der therapeutische Wirkstoff verabreicht wird, und die zweite Periode ein Zeitraum von 46 bis 54 Wochen ist, in dem der therapeutische Wirkstoff verabreicht wird.

12. Das Verfahren nach Anspruch 6, umfassend einen Schritt, bei dem das Subjekt auf der Grundlage der Expressionsniveaus des MS4A4A-Gens von Patienten mit rheumatoider Arthritis, gesunden Individuen sowie Patienten mit einer anderen Erkrankung als rheumatoider Arthritis unterschieden wird.

## Revendications

1. Méthode pour prédire l'efficacité d'un agent thérapeutique contre la polyarthrite rhumatoïde pour un patient atteint de polyarthrite rhumatoïde, la méthode comprenant les étapes de :
mesurer le niveau d'expression de MS4A4A dans le sang périphérique prélevé chez le patient préalablement au début d'administration de l'agent thérapeutique contre la polyarthrite rhumatoïde ; et
mesurer le niveau d'expression de MS4A4A dans le sang périphérique prélevé chez le patient après une première période d'administration de l'agent thérapeutique contre la polyarthrite rhumatoïde ;
dans laquelle une décroissance du niveau d'expression du MS4A4A après la première période indique une décroissance de valeur numérique d'un indice associé avec la polyarthrite rhumatoïde chez le patient après la seconde période d'administration de l'agent thérapeutique.

2. Méthode selon la revendication 1, dans laquelle la première période est une période allant jusqu'à quatre semaines d'administration de l'agent thérapeutique.

3. Méthode selon la revendication 1, dans laquelle la seconde période est une période de 12 semaines à 54 semaines d'administration de l'agent thérapeutique.

4. Méthode selon la revendication 1, dans laquelle l'agent thérapeutique est choisi parmi le groupe constitué du tocilizumab, du méthotrexate, et des inhibiteurs du facteur de nécrose tumorale.

5. Méthode selon la revendication 1, dans laquelle l'indice est choisi parmi le groupe constitué de DAS28-CRP, DAS28-ESR, DAS44, critères de réponse EULAR, CDAI, SDAI, critères de réponse ACR, et le niveau de CRP.

6. Méthode pour estimer une valeur numérique d'un indice d'activité de maladie associé avec la polyarthrite rhumatoïde chez un sujet, la méthode comprenant les étapes de :
mesurer le niveau d'expression de MS4A4A dans le sang périphérique prélevé chez le sujet ;
analyser le niveau d'expression de MS4A4A mesuré en utilisant le profil d'expression génique préparé à l'avance corrélé avec l'indice ; et
estimer une valeur numérique pour l'indice chez le sujet en fonction des résultats de l'analyse ;
l'étape d'analyse étant effectuée en comparant le niveau d'expression de MS4A4A mesuré avec un profil d'expression génique indiquant la corrélation entre le niveau d'expression de MS4A4A et l'indice, le profil étant produit en fonction des niveaux d'expression du gène MS4A4A dans le sang périphérique provenant de patients de polyarthrite rhumatoïde et l'indice d'activité de maladie du patient de polyarthrite rhumatoïde, où l'indice est choisi parmi le groupe constitué de DAS28-CRP, DAS28-ESR, DAS44, critères de réponse EULAR, CDAI, SDAI, critères de réponse ACR, et le niveau de CRP.

7. Méthode selon la revendication 6, dans laquelle l'indice indique l'activité de maladie de polyarthrite rhumatoïde, ou évalue des effets thérapeutiques l'agent thérapeutique contre la polyarthrite rhumatoïde.

8. Méthode selon la revendication 6, dans laquelle une corrélation positive entre le profil d'expression génique et un niveau élevé d'expression du gène MS4A4A dans le sang périphérique prélevé chez le sujet indique un niveau élevé d'activité de maladie.

9. Méthode selon la revendication 6, dans laquelle le sujet est atteint de polyarthrite rhumatoïde.

10. Méthode selon la revendication 9, dans laquelle l'étape de mesure consiste à mesurer le niveau d'expression de MS4A4A dans le sang périphérique prélevé chez un patient préalablement au et après le début d'administration de l'agent thérapeutique contre la polyarthrite au sujet, et les niveaux d'expression de MS4A4A dans le sang périphérique prélevé chez un patient préalablement à et après le début d'administration sont analysés en utilisant le profil d'expression génique pour évaluer les effets thérapeutiques de l'agent thérapeutique.

11. Méthode selon la revendication 1, dans laquelle la première période est une période allant jusqu'à quatre semaines d'administration de l'agent thérapeutique et la seconde période est une période de 46 à 54 semaines d'administration de l'agent thérapeutique.

12. Méthode selon la revendication 6, comprenant une étape de distinguer le sujet de patients de polyarthrite rhumatoïde et de individus en bonne santé et un patient souffrant d'une maladie autre que la polyarthrite rhumatoïde, en fonction des niveaux d'expression du gène MS4A4A.
